# EUROPÄISCHE PATENTANMELDUNG

(11) **EP 4 512 798 A1**
(43) Veröffentlichungstag der Anmeldung: **26.02.2025**
(21) Anmeldenummer: 23193109.8
(22) Anmeldetag: 24.08.2023
(51) Int. Cl.: C07C 263/20, B01D 3/00, C07B 63/00

(54) **VERFAHREN ZUR DIISOCYANAT-DESTILLATION**

(71) Anmelder: Covestro Deutschland AG, 51373 Leverkusen (DE)
(72) Erfinder: Merkel, Michael, 40223 Düsseldorf (DE); Schneider, Ralf, 51375 Leverkusen (DE); Krieger, Alexandra, 51065 Köln (DE); Ehrig, Martin, 51373 Leverkusen (DE); Leimbrink, Mathias, 44139 Dortmund (DE)
(74) Vertreter: Levpat

(57) **Zusammenfassung**

Die vorliegende Erfindung betrifft ein Verfahren zur Diisocyanat-Destillation, umfassend die Schritte A) Bereitstellen eines Destillationssystems, B) Destillation einer Zusammensetzung, enthaltend im Wesentlichen Pentamethylendiisocyanat, in dem Destillationssystem, dadurch gekennzeichnet, dass nach Schritt B) der Schritt C) Destillation einer Zusammensetzung, enthaltend im Wesentlichen ein Diisocyanat verschieden von Pentamethylendiisocyanat, in dem Destillationssystem unter Erhalt eines Destillats durchgeführt wird.

## Beschreibung

Die Erfindung betrifft ein Verfahren zur Diisocyanat-Destillation, umfassend eine Reinigung des Destillationssystems sowie ein Destillationssystem zur Durchführung des Verfahrens und eine Verwendung bestimmter Diisocyanate zur Reinigung eines Destillationssystems, welches für die Destillation von Pentamethylendiisocyanat verwendet wurde.

Pentamethylendiisocyanat (im Weiteren Text auch als PDI, 1,5-PDI oder auch 1,5-Pentandiisocyanat bezeichnet) ist ein wertvoller Rohstoff für die die Herstellung von Polyurethanen. Die Herstellung von PDI erfolgt technisch vorteilhaft durch Phosgenierung von Pentamethylendiamin (PDA). Die Umsetzung kann dabei sowohl in kondensierter Phase (Flüssigphasenphosgenierung, im weiteren Text auch als FPP bezeichnet) als auch in der Gasphase (Gasphasenphosgenierung, im weiteren Text auch als GPP bezeichnet) erfolgen. Nach der Phosgenierungsreaktion fällt das PDI üblicherweise als im Wesentlichen flüssiger Rohproduktstrom an, aus dem das PDI in einer mehrstufigen Destillationssequenz isoliert wird.

Ein bislang ungelöstes Problem bei der Destillation von monomeren Diisocyanaten ist deren Tendenz, beim längeren Betrieb der Destillationssysteme Ablagerungen zu bilden. Diese können in verschiedenen Bereichen des Destillationssystems auftreten und erfordern von Zeit zu Zeit Produktionsunterbrechungen, um die Destillationssysteme zu reinigen oder Einbauten zu ersetzen.

EP 3 533 785 A1 beschreibt eine Methode zur Herstellung von 1,5-PDI, wobei die Destillation in einer mehrstufigen Destillationssequenz erfolgt. Als Reinigungsschritte sind eine Lösungsmittelabtrennung, eine Wärmebehandlung, eine Rückstandsabtrennung und eine Reindestillation offenbart. Probleme durch Ablagerungen beim längeren Betrieb der Destillation werden nicht behandelt.

In der WO2017/076551 A1 wird eine Trennwandkolonne beschrieben, die sich für die Destillation von Isocyanaten wie beispielsweise TDI, MDI, HDI, PDI, Isophorondiisocyanat (IPDI), XDI oder Dicyclohexylmethandiisocyanat (H₁₂MDI) eignet, wobei das gewünschte Isocyanat jeweils als Seitenstrom entnommen wird, während leichtsiedende Komponenten am Kopf der Kolonne anfallen. Probleme durch Ablagerungen beim längeren Betrieb des Destillationssystems werden auch hier nicht behandelt.

US 6,550,485 B2 beschreibt eine Methode zur Reinigung von Destillationssystemen, die polymerisierbare stickstoffhaltige Verbindungen beziehungsweise aus derartigen Verbindungen gebildete Ablagerungen beinhalten. Die Methode besteht im Spülen des Destillationssystems mit einer sauren Flüssigkeit, vorzugsweise mit organischen oder anorganischen Säuren in wässriger Lösung. Bevorzugt erfolgt im Anschluss eine weitere Spülung des Destillationssystems mit Wasser, um die Säure aus dem System zu entfernen. Auch wenn es sich bei Diiscoyanaten im Allgemeinen und Pentamethylendiisocyanat im Speziellen um polymerisierbare stickstoffhaltige Verbindungen handelt, ist die offenbarte Methode für Destillationssysteme, die zur Destillation von Diisocyanaten genutzt werden, gänzlich ungeeignet. Aufgrund der bekannten Reaktivität von Diisocyanaten oder auch von bereits gebildeten Oligomeren, würde diese Methode die Bildung weiterer schwer löslicher Ablagerungen, z.B. in Form von Isocyanuraten oder Harnstoffen, nach sich ziehen und so die Problematik noch verschlimmern.

US 7,182,839 B2 offenbart eine Methode, mit der sich bei Destillation, insbesondere der Lösungsmittelabtrennung, von fouling-anfälligen Medien die Bildung Ablagerungen unterdrücken lässt. Als fouling-anfällige Medien werden z.B. auch Diisocyanate angeführt. Die Methode erfordert die Anwesenheit von N,N`-Dialkylamiden oder Fettsäuren, die für eine Dispergierung on festen oder halbfesten Partikeln sorgen sollen, ehe sich diese Ablagern können. Diese Methode erfordert das Einbringen und Entfernen ansonsten Prozessfremder Stoffe in das Destillationssystem und bedingt damit einen erheblichen zusätzlichen Aufwand. Auch lassen sich nicht alle Bereiche eines Diisocyanat-Destillationssystems auf diese Weise schützen, ohne zwangsläufig auch eine Kontamination des destillierten Produkts zu verursachen.

Es bestand also ein Bedarf an einem Verfahren zur Destillation von Diisocyanat-Monomeren, bei dem eine Reinigung des Destillationssystems ohne die üblicherweise damit verbundenen Nachteile erfolgen kann.

Überraschend wurde gefunden, dass diese Aufgabe für bestimmte Diisocyanate gelöst wird durch ein Verfahren zur Diisocyanat-Destillation, umfassend die Schritte
A) Bereitstellen eines Destillationssystems und
B) Destillation einer ersten Zusammensetzung, enthaltend im Wesentlichen Pentamethylendiisocyanat, in dem Destillationssystem, dadurch gekennzeichnet, dass nach Schritt B) der Schritt
C) Destillation einer zweiten Zusammensetzung, enthaltend im Wesentlichen ein Diisocyanat verschieden von Pentamethylendiisocyanat (PDI), in dem Destillationssystem unter Erhalt eines Destillats durchgeführt wird.

Unter einer Diisocyanat-Destillation wird vorliegend ein thermisches Trennverfahren verstanden, bei dem ein monomeres Diisocyanat wenigstens einmal verdampft und wieder kondensiert wird, um es von anderen, insbesondere höher siedenden Verunreinigungen abzutrennen und in hoher Reinheit zu gewinnen. Der Begriff umfasst insbesondere die wiederholte Verdampfung und Kondensation unter Verwendung einer Destillationskolonne mit einer Mehrzahl an Trennstufen. Derartige Verfahren werden auch als Rektifikation bezeichnet und sind vorliegend, sofern nicht ausdrücklich anders angegeben, von den Begriffen Destillation bzw. Diisocyanat-Destillation mit umfasst.

Ein Destillationssystem umfasst vorliegend wenigstens eine Destillationskolonne sowie die damit verbundenen Peripheriegeräte wie beispielsweise Verdampfer, Wärmetauscher, Kondensatoren, Pumpen und die Rohrleitungen, die diese miteinander verbinden. Die Destillationskolonne kann mit allen dem Fachmann bekannten Einrichtungen zur Erhöhung der Trennleistung ausgestattet sein. Dazu zählen insbesondere Schüttungen, Packungen und Böden verschiedener Ausgestaltung.

Es zeigte sich bei der Destillation von 1,5-Pentandiisocyanat, in einem Destillationssystem bestehend aus einer Kolonne mit Seitenabnahme für das destillierte Produkt, einem Kopfkondensator und einem Umlaufverdampfer, dass bei längerer Betriebsdauer an verschiedenen Stellen des Destillationssystems Ablagerungen auftreten können, die sich negativ auf den Betrieb des Destillationssystems auswirken. Insbesondere an den Kühlflächen des Destillationssystems, also den Wärmeaustauschflächen des Kopfkondensators, traten weiße bis hellgelbe Ablagerungen auf, die den Wärmeübergang beeinträchtigten und eine Reinigung erforderlich machten. Diese Ablagerungen konnten wiederholt beobachtet werden und verschiedene Konsistenz von pulverartig bis hin zu gelee- oder schaumartig aufweisen.

Überraschend wurde nun gefunden, dass ein Wechsel der zu destillierenden Rohware von der ersten Zusammensetzung enthaltend im Wesentlichen PDI auf eine zweite Zusammensetzung enthaltend im Wesentlichen ein Diisocyanat verschieden von PDI, mit einem reinigenden Effekt einhergeht, wobei nicht nur gegebenenfalls verbliebenes Pentandiisocyanat in der zweiten Zusammensetzung gelöst und letztlich ausgetragen wird, sondern auch die durch die PDI-Destillation verursachten Ablagerungen deutlich reduziert oder sogar aufgelöst werden. Diese Reinigung war ausreichend, um das Destillationssystem über längere Zeit weiter betreiben zu können. Es musste nicht komplett außer Betrieb genommen werden, um es für eine mechanische Reinigung, beispielsweise eine Hochdruckreinigung, oder gar für den Austausch von Teilen des Destillationssystems, wie beispielsweise der Packungen, zu öffnen.

In einer bevorzugten Ausführungsform enthält die zweite Zusammensetzung in Schritt C) wenigstens 80 Gew.-%, vorzugsweise wenigstens 90 Gew.-% und besonders bevorzugt wenigstens 95 Gew.-% des Diisocyanats verschieden von Pentamethylendiisocyanat. Bevorzugt ist das Diisocyanat ausgewählt aus der Gruppe umfassend oder bestehend aus Hexamethylendiisocyanat, Isophorondiisocyanat und 4,4`-Diisocyanatodicyclohexylmethan, besonders bevorzugt ausgewählt aus der Gruppe umfassend oder bestehend aus Hexamethylendiisocyanat und Isophorondiisocyanat und ganz besonders bevorzugt ist das Diisocyanat Hexamethylendiisocyanat.

In einer bevorzugten Ausführungsform wurde das Pentamethylendiisocyanat durch Phosgenierung von Pentamethylendiamin, bevorzugt durch Phosgenierung von biobasiertem Pentamethylendiamin, hergestellt und/oder das Diisocyanat verschieden von PDI wurde durch Phosgenierung des entsprechenden Diamins hergestellt.

In einer besonderes bevorzugten Ausführungsform erfolgt die Phosgenierung des Diamins verschieden von Pentamethylendiamin wenigstens zum Teil, bevorzugt vollständig in der derselben Vorrichtung wie zuvor die Phosgenierung von Pentamethylendiamin. Es handelt sich bei der Phosgeniervorrichtung in dieser Ausführungsform also um eine Multi-Purpose-Phosgeniervorrichtung, die dazu eingerichtet ist, 1,5-Diaminopentan sowie wenigstens ein Diamin vorzugsweise ausgewählt aus der Gruppe bestehend aus 1.6-Diaminohexan, Isophorondiamin und Bis(4-Aminocylohexyl)methan kampagnenweise mit Phosgen zu den entsprechenden Isocyanaten umzusetzen. Diese Ausführungsform hat den Vorteil, dass Skaleneffekte genutzt werden können, auch wenn nur ein begrenzter Bedarf an den einzelnen Isocyanaten vorhanden ist. Durch den kampagnenweisen Betrieb lässt sich der dieser Erfindung zugrundeliegende Reinigungseffekt besonders einfach nutzen.

In einer bevorzugten Ausführungsform des Verfahrens werden Inhalte des Destillationssystems zwischen den Schritten B) und C) zumindest teilweise, bevorzugt überwiegend in einen ersten Ablassbehälter transferiert und/oder es werden Inhalte des Destillationssystems nach Schritt C) zumindest teilweise, bevorzugt überwiegend in einen zweiten Ablassbehälter transferiert.

Diese Ausführungsform hat den Vorteil, dass das Destillat, welches in Schritt C) erhalten wird, schon von Anfang an nur einen sehr geringen Gehalt an PDI aufweist und die Zeit, bis es vollständig frei von PDI anfällt, verkürzt wird und/oder dass ein gegebenenfalls im Anschluss erzeugtes PDI Destillat schon von Anfang an nur einen sehr geringen Gehalt an von PDI verschiedenem Diisocyanat aus Schritt C) aufweist und die Zeit, bis es vollständig frei von diesem anfällt, verkürzt wird.

Es ist grundsätzlich auch möglich, in einem weiteren Schritt zumindest Teile des Destillationssystems mit einem organischen Lösungsmittel zu spülen. Dieses Lösungsmittel sollte sich chemisch gegenüber Isocyanat-Gruppen weitgehend inert verhalten. Bei den geeigneten organischen Lösemitteln handelt es sich im Rahmen der vorliegenden Erfindung nicht um eines der in Schritt B) oder C) beteiligten Isocyanate.

Geeignete organische Lösungsmittel wären beispielsweise aromatische Kohlenwasserstoffe, bevorzugte Lösungsmittel sind Chlorbenzol, o-Dichlorbenzol, p-Dichlorbenzol, Trichlorbenzole, die entsprechenden Chlortoluole oder Chlorxylole, Chlorethylbenzol, Monochlordiphenyl, α-bzw. β-Naphthylchlorid, Benzoesäureethylester, Phthalsäuredialkylester, Diisodiethylphthalat, Toluol, Xylole oder deren Mischungen, besonders bevorzugte Lösungsmittel sind Chlorbenzol, o-Dichlorbenzol, p-Dichlorbenzol, Toluol, Xylol oder Mischungen von diesen und ganz besonders bevorzugte Lösungsmittel sind Chlorbenzol und/oder o-Dichlorbenzol. Es ist dabei allerdings zu beachten, dass eine solche Spülung großen Aufwand für die Bereitstellung, Aufbereitung und Entsorgung des Lösungsmittels verursacht und eine Kontamination des später erzeugten Destillats ist nicht immer vollständig vermeidbar ist. Deshalb ist es besonders bevorzugt, dass zwischen den Schritten B) und C) kein wesentliches, bevorzugt gar kein Spülen des Destillationssystems mit einem organischen Lösungsmittel und/oder mit einer wässrigen Lösung als Spülmittel erfolgt. Das Spülen des Destillationssystems wird vorliegend dann als nicht wesentlich angesehen, wenn die anfallende Spüllösung vorzugsweise weniger als 50 Gewichts-% des Spülmittels, stärker bevorzugt weniger als 30 Gew.-% des Spülmittels und besonders bevorzugt weniger als 10 Gew.-% des Spülmittels, enthält oder wenn vorzugsweise nur bis zu zwei einzelne Apparate des Destillationssystems vom Rest des Systems gespült, bevorzugt isoliert und dann gespült, werden.

In Folge wartungsbedingter Stillstände, bei denen Apparate des Destillationssystems geöffnet werden, kann es zum Eintrag von Luftfeuchtigkeit in das Destillationssystem kommen. Diese kann im weiteren Verlauf des Verfahrens mit Isocyanat reagieren und zu schwer löslichen Harnstoffablagerungen auf den Oberflächen des Destillationssystems, die im Kontakt zu den Prozessmedien stehen, führen. In einer weiteren bevorzugten Ausführungsform umfasst das Verfahren deshalb als weiteren Schritt A1) eine Trocknung von zumindest Teilen des Destillationssystems. Vorzugsweise erfolgt die Trocknung durch das Anlegen eines Unterdrucks. Besonders bevorzugt werden die zu trocknenden Teile des Destillationssystems mehrfach evakuiert und danach mit trockenem Inertgas belüftet. Die vorgenannte Trocknung kann bei Bedarf zu beliebigen Zeitpunkten, also beispielsweise auch nach Schritt B) oder C) erfolgen.

In einer weiteren Ausführungsform umfasst der Schritt C) folgende Teilschritte:
c1) Andestillieren einer Zusammensetzung enthaltend im Wesentlichen ein Diisocyanat, ausgewählt aus der Gruppe bestehend aus Hexamethylendiisocyanat, Isophorondiisocyanat und 4,4`-Diisocyanatodicyclohexylmethan, in dem mindestens einen Destillationssystem,
c2) Transferieren zumindest eines Teilinhalts des Destillationssystems in einen zweiten Ablassbehälter,
c3) kontinuierliche Destillation einer Zusammensetzung enthaltend im Wesentlichen das gleiche Diisocyanat wie die Zusammensetzung in Schritt (c1) in dem Destillationssystem unter Erhalt eines Destillats und
c4) Optional und gegebenenfalls nach dem Durchlaufen wenigstens eines Aufbereitungsschrittes, verschneiden des in Teilschritt c2) transferierten Teilinhalts aus dem Ablassbehälter durch Einbringen in die kontinuierliche Destillation in Teilschritt c3) oder das in Teilschritt c3) erhaltene Destillat.

Unter dem Andestillieren ist vorliegend zu verstehen, dass die entsprechende Zusammensetzung in das Destillationssystem eingebracht und dort durch Einbringen von Wärmeenergie zumindest teilweise verdampft wird. Die entstehenden Brüden werden kondensiert und vorzugsweise vollständig als Rücklauf zurückgeführt. Alternativ, aber weniger bevorzugt, kann ein Teil oder auch die Gesamtheit der kondensierten Brüden aus dem Destillationssystem entfernt und entsorgt werden. Der Vorteil dieser Ausführungsform liegt darin, das Destillationssystem durch das Andestillieren bereits mit dem folgenden Diisocyanat zu beaufschlagen und ggfs. vorhandene Reste des Pentamethylendiisocyanats effektiv aus dem System zu entfernen. Sofern der optionale Schritt c4) ausgeführt wird, können Spülverluste vermieden werden und so die Produktausbeute optimiert werden. Es ist dabei darauf zu achten, dass der zu verschneidende Mengenstrom nur so groß gewählt wird, dass die Produktspezifikation des im Schritt C) zu destillierenden Diisocyanats möglichst zu jeder Zeit eingehalten wird.

Die reinigende Wirkung des Schritts C) auf das Destillationssystems lässt sich am besten ausnutzen, indem das Destillationssystem letztlich dazu genutzt wird, im Wechsel eine Zusammensetzung enthaltend im Wesentlichen PDI und eine Zusammensetzung enthaltend ein Diisocyanat verschieden von PDI zu destillieren. Dabei kann sogar für längere Zeiträume auf den Einsatz von beispielsweise Lösungsmitteln zum Spülen des Destillationssystems verzichtet werden und Ausfallzeiten durch sonst erforderliche Reinigungsphasen entfallen, da die reinigende Wirkung erzielt wird, indem ebenfalls ein Diisocyanat als Wertprodukt destilliert wird. In einer bevorzugten Ausführungsform des Verfahrens wird also nach Schritt C) erneut eine Zusammensetzung enthaltend im Wesentlichen Pentamethylendiisocyanat in dem Destillationssystem destilliert und dabei ist es besonders bevorzugt, zwischen den Schritten C) und der erneuten Destillation einer Zusammensetzung enthaltend im Wesentlichen Pentamethylendiisocyanat nach dem Schritt C) kein wesentliches, bevorzugt gar kein Spülen des Destillationssystems mit einem organischen Lösungsmittel und/oder mit einer wässrigen Lösung erfolgt.

In einer weiteren Ausführungsform betrifft die Erfindung ein Verfahren zur Diisocyanat-Destillation, umfassend die Schritte
A) Bereitstellen eines Destillationssystems
B) Destillation einer Zusammensetzung enthaltend im Wesentlichen Pentamethylendiisocyanat in dem Destillationssystem,
   dadurch gekennzeichnet, dass nach Schritt B) der Schritt
C) Destillation einer Zusammensetzung enthaltend im Wesentlichen ein Diisocyanat verschieden von Pentamethylendiisocyanat in dem Destillationssystem unter Erhalt eines Destillats durchgeführt wird
und nach Schritt C) die Schritte B) und C) vorzugsweise ein oder mehrere Male im Wechsel wiederholt werden. Für dieses Verfahren gelten die genannten Ausführungsformen und Bevorzugungen gleichermaßen.

Ein weiterer Gegenstand der Erfindung ist ein Diisocyanat-Destillationssystem zur Durchführung des Verfahrens gemäß den Ansprüchen 1 bis 12, umfassend oder bestehend aus
(a) mindestens einer Zulaufvorrichtung zum Einbringen von Diisocyanat-Zusammensetzungen in das Diisocyanat-Destillationssystem,
(b) einem Sumpfbehälter mit zugeordneter Fraktionierkolonne mit oder ohne Trennwand;
(c) mindestens einem Verdampfer zum zumindest teilweisen Verdampfen wenigstens eines Teils des Inhalts aus dem Sumpfbehälter
(d) einer Vakuumvorrichtung zur Senkung des Innendrucks in der Kolonne
(e) mindestens einem Kondensator zur zumindest teilweisen Kondensation der Brüden aus der Fraktionierkolonne
(f) mindestens zwei Ablassbehältern, die dazu eingerichtet sind, dass zumindest Teilinhalte des Diisocyanat-Destillationssystems in sie transferiert werden,
(g) eine Entnahmevorrichtung, die dazu eingerichtet ist, destilliertes Produkt aus dem Diisocyanat-Destillationssystem auszuschleusen und/oder in die Fraktionierkolonne zurückzuführen
(h) optional mindestens einer Fördereinrichtung zur Förderung des Sumpfinhalts zu dem mindestens einen Verdampfer,
(i) optional mindestens einer Transporteinrichtung, die dazu eingerichtet ist, Inhalte aus einem der Ablassbehälter mit einem bereits destillierten Produkt aus dem Diisocyanat-Destillationssystem zu verschneiden und/oder Inhalte aus einem der Ablassbehälter derart zurückzuführen, dass sie erneut die Fraktionierkolonne durchlaufen und/oder aus dem Diisocyanat-Destillationssystem zu entnehmen.

Ein weiterer Gegenstand der Erfindung ist die Verwendung einer Zusammensetzung enthaltend im Wesentlichen ein Diisocyanat, ausgewählt aus der Gruppe umfassend oder bestehend aus Hexamethylendiisocyanat, Isophorondiisocyanat und 4,4`-Diisocyanatodicyclohexylmethan, zur Reinigung mindestens eines Destillationssystems, welches für die Destillation mindestens einer Zusammensetzung enthaltend im Wesentlichen Pentamethylendiisocyanat genutzt wurde.

Im Folgenden wird die Erfindung an Beispielen näher beschrieben, ohne jedoch auf diese beschränkt zu sein.

### Ausführungsbeispiel

Eine zuvor von Lösungsmitteln befreite Rohware aus der Phosgenierung von 1,5-Pentandiamin wurde in einer Versuchsanlage kontinuierlich destilliert. Diese Rohware bestand zu ca. 98 Gew.-% aus Pentamethylendiisocyanat, den Rest zu 100% machten leicht- und schwersiedende Nebenkomponenten aus. Die Destillation erfolgte bei einem Kopfdruck in der Kolonne von 16 mbar. Am Kopf der Kolonne wurden die gasförmigen Brüden entnommen und in einem Kondensator niedergeschlagen. Ein kleiner Teil des Kondensats wurde entnommen, um Leichtsieder abzutrennen, der Rest wurde als Rücklauf in die Kolonne gegeben. Im Seitenstrom wurde Pentamethylendiisocyanat mit einer Reinheit von 99,8 Gew.-% entnommen und am Sumpf wurde ein Konzentrat der schwersiedenden Nebenkomponenten in Pentamethylendiisocyanat ausgeschleust.

Nach einer Woche zeigte sich an den Kühlflächen des Kopf-Kondensators ein weißer, teilweise gelblicher Belag. Die Destillation wurde nach 3 Wochen unterbrochen und die in der Versuchsanlage verbliebenen Reste wurden in einen Ablassbehälter abgelassen.

Anschließend wurde, ohne die Destillationsanlage zu spülen, die Rohware auf eine ebenfalls bereits von Lösungsmittel befreite Rohware aus der Phosgenierung von 1,6-Hexandiamin gewechselt und die Destillation erneut gestartet. Dabei wurde beobachtet, dass sich die Beläge an den Kühlflächen teilweise auflösten.

## Patentansprüche

1. Verfahren zur Diisocyanat-Destillation, umfassend die Schritte
A) Bereitstellen eines Destillationssystems
B) Destillation einer Zusammensetzung, enthaltend im Wesentlichen Pentamethylendiisocyanat, in dem Destillationssystem,
**dadurch gekennzeichnet, dass** nach Schritt B) der Schritt
C) Destillation einer Zusammensetzung, enthaltend im Wesentlichen ein Diisocyanat verschieden von Pentamethylendiisocyanat, in dem Destillationssystem unter Erhalt eines Destillats
durchgeführt wird.

2. Verfahren gemäß Anspruch 1, **dadurch gekennzeichnet, dass** die Zusammensetzung in Schritt C) wenigstens 80 Gew.-%, vorzugsweise wenigstens 90 Gew.-% und besonders bevorzugt wenigstens 95 Gew.-% des Diisocyanats verschieden von Pentamethylendiisocyanat enthält.

3. Verfahren gemäß einem der Ansprüche 1 oder 2, **dadurch gekennzeichnet, dass** das Diisocyanat verschieden von PDI ausgewählt ist aus der Gruppe umfassend oder bestehend aus Hexamethylendiisocyanat, Isophorondiisocyanat und 4,4`-Diisocyanatodicyclohexylmethan, bevorzugt ausgewählt ist aus der Gruppe umfassend oder bestehend aus Hexamethylendiisocyanat und Isophorondiisocyanat und besonders bevorzugt Hexamethylendiisocyanat ist.

4. Verfahren gemäß einem der Ansprüche 1 bis 3, **dadurch gekennzeichnet, dass** das Pentamethylendiisocyanat durch Phosgenierung von Pentamethylendiamin, bevorzugt durch Phosgenierung von biobasiertem Pentamethylendiamin, hergestellt wurde und/oder das Diisocyanat verschieden von Pentamethylendiisocyanat durch Phosgenierung des entsprechenden Diamins hergestellt wurde.

5. Verfahren gemäß Anspruch 4, **dadurch gekennzeichnet, dass** die Phosgenierung des Diamins verschieden von Pentamethylendiamin wenigstens zum Teil in der derselben Vorrichtung erfolgt wie zuvor die Phosgenierung von Pentamethylendiamin.

6. Verfahren gemäß einem der Ansprüche 1 bis 5, **dadurch gekennzeichnet, dass** Inhalte des Destillationssystems zwischen den Schritten B) und C) zumindest teilweise in einen ersten Ablassbehälter transferiert werden und/oder dass Inhalte des Destillationssystems nach Schritt C) zumindest teilweise in einen zweiten Ablassbehälter transferiert werden.

7. Verfahren gemäß einem der Ansprüche 1 bis 6, **dadurch gekennzeichnet, dass** das Destillationssystem zwischen den Schritten B) und C) kein wesentliches, bevorzugt gar kein Spülen des Destillationssystems mit einem organischen Lösungsmittel und/oder mit einer wässrigen Lösung erfolgt.

8. Verfahren gemäß einem der Ansprüche 1 bis 7, **dadurch gekennzeichnet, dass** das Verfahren einen weiteren Schritt
A1) Trocknen von zumindest Teilen des Destillationssystems umfasst.

9. Verfahren gemäß einem der Ansprüche 1 bis 8, **dadurch gekennzeichnet, dass** Schritt C) folgende Teilschritte umfasst
c1) Andestillieren einer Zusammensetzung enthaltend im Wesentlichen ein Diisocyanat, ausgewählt aus der Gruppe bestehend aus Hexamethylendiisocyanat, Isophorondiisocyanat und 4,4`-Diisocyanatodicyclohexylmethan, in dem mindestens einen Destillationssystem,
c2) Transferieren zumindest eines Teilinhalts des Destillationssystems in einen zweiten Ablassbehälter,
c3) Kontinuierliche Destillation einer Zusammensetzung enthaltend im Wesentlichen das gleiche Diisocyanat wie die Zusammensetzung in Schritt (c1) in dem Destillationssystem unter Erhalt eines Destillats und
c4) Optional und gegebenenfalls nach dem Durchlaufen wenigstens eines Aufbereitungsschrittes, verschneiden des in Teilschritt c) transferierten Inhalts aus dem Ablassbehälter durch Einbringen in die kontinuierliche Destillation in Teilschritt c3) oder das in Teilschritt c3) erhaltene Destillat.

10. Verfahren gemäß einem der Ansprüche 1 bis 9, **dadurch gekennzeichnet, dass** nach Schritt C) erneut eine Zusammensetzung enthaltend im Wesentlichen Pentamethylendiisocyanat in dem Destillationssystem destilliert wird.

11. Verfahren gemäß Anspruch 10, **dadurch gekennzeichnet, dass** zwischen den Schritten C) und der erneuten Destillation einer Zusammensetzung enthaltend im Wesentlichen Pentamethylendiisocyanat nach dem Schritt C) kein wesentliches, bevorzugt gar kein Spülen des Destillationssystems mit einem organischen Lösungsmittel und/oder mit einer wässrigen Lösung erfolgt.

12. Diisocyanat-Destillationssystem zur Durchführung des Verfahrens gemäß den Ansprüchen 1 bis 12, umfassend oder bestehend aus
(a) mindestens einer Zulaufvorrichtung zum Einbringen von Diisocyanat-Zusammensetzungen in das Diisocyanat-Destillationssystem,
(b) einem Sumpfbehälter mit zugeordneter Fraktionierkolonne mit oder ohne Trennwand;
(c) mindestens einem Verdampfer zum zumindest teilweisen Verdampfen wenigstens eines Teils des Inhalts aus dem Sumpfbehälter
(d) einer Vakuumvorrichtung zur Senkung des Innendrucks in der Kolonne
(e) mindestens einem Kondensator zur zumindest teilweisen Kondensation der Brüden aus der Fraktionierkolonne
(f) mindestens zwei Ablassbehältern, die dazu eingerichtet sind, dass zumindest Teilinhalte des Diisocyanat-Destillationssystems in sie transferiert werden,
(g) eine Entnahmevorrichtung, die dazu eingerichtet ist, destilliertes Produkt aus dem Diisocyanat-Destillationssystem auszuschleusen und/oder in die Fraktionierkolonne zurückzuführen
(h) optional mindestens einer Fördereinrichtung zur Förderung des Sumpfinhalts zu dem mindestens einen Verdampfer,
(i) optional mindestens einer Transporteinrichtung, die dazu eingerichtet ist, Inhalte aus einem der Ablassbehälter mit einem bereits destillierten Produkt aus dem Diisocyanat-Destillationssystem zu verschneiden und/oder Inhalte aus einem der Ablassbehälter derart zurückzuführen, dass sie erneut die Fraktionierkolonne durchlaufen und/oder aus dem Diisocyanat-Destillationssystem zu entnehmen.

13. Verwendung einer Zusammensetzung enthaltend im Wesentlichen ein Diisocyanat, ausgewählt aus der Gruppe umfassend oder bestehend aus Hexamethylendiisocyanat, Isophorondiisocyanat und 4,4`-Diisocyanatodicyclohexylmethan, zur Reinigung mindestens eines Destillationssystems, welches für die Destillation mindestens einer Zusammensetzung enthaltend im Wesentlichen Pentamethylendiisocyanat genutzt wurde.
